# EUROPEAN PATENT APPLICATION

(11) **EP 3 718 512 A1**
(43) Date of publication of application: **07.10.2020**
(21) Application number: 19167259.1
(22) Date of filing: 04.04.2019
(51) Int. Cl.: A61F 2/38

(54) **TIBIA CONDYLE RESURFACING PROSTHESIS**

(71) Applicant: Kyon AG, 8005 Zürich (CH)
(72) Inventor: Tepic, Slobodan, CH-8057 Zurich (CH); Bresina, Stephen, CH-7260 Davos Dorf (CH)
(74) Representative: Weickmann & Weickmann PartmbB

(57) **Abstract**

The present invention resolves the main problems of bone-sparing resurfacing surgery of the medial compartment of the knee in dogs and in people. With a hard, low abrasion, low friction coating of the implant (100), such as ADLC or pyrolytic carbon, it is possible to resurface only one side of the joint. In this case, the tibia plateau resurfacing with a concave recess (20) on its femur-facing surface provides not only pain-free mobility but also the necessary stability of the joint with a deficient cruciate ligament. The planar osteotomy of the medial condyle, with or without resurfacing, can also be used to emulate TPLO procedure limited to the medial compartment of the stifle.

## Description

The present invention relates to a medial tibial condyle resurfacing implant for hemiarthroplasty of the knee joint, for use in human and veterinary medicine. Further, the present invention relates to a medial tibial condyle osteotomy emulating procedure for stabilization of the cranial cruciate deficient stifle in human and animal knees.

### Background

In people but also in dogs, the medial condyles of the tibia and the femur are at high risk of degenerative arthritis as - among other possible causes - a consequence of cruciate ligament rupture or of skeletal deformities resulting in increased loading of the medial knee compartment.

In human orthopedics, the usual approach is to perform either a total knee replacement or, increasingly, to replace only smaller areas of the affected joint.

In human orthopedics, the medial compartment of the knee is also commonly affected by overloading due to varus deformity of the leg. If the cartilage is still able to support the load, the preferred surgical intervention is medial opening-wedge osteotomy of the proximal tibia. If the cartilage is lost in degeneration the usual intervention is either total knee prosthesis or a partial replacement, e.g. with the femoral medial condyle metal implant articulating on the metal-backed polyethylene tibial component.

In veterinary orthopedics, the only currently available solution is the total knee prosthesis. Due to high costs and complicated surgical execution, very few dogs are being operated with a total knee.

In the veterinary orthopedic surgery, one of the most commonly treated conditions is the rupture of the Cranial Cruciate Ligament (CrCL). There are credible estimates putting the number of cases treated annually in the US at about one million. About 10% of those interventions are so-called geometry modifying surgeries, most common of which are Tibial Plateau Leveling Osteotomy (TPLO) and Tibial Tuberosity Advancement (TTA). The remaining 90% are treated by different versions of extra-capsular sutures aimed at providing some stability to the joint but those, in all cases, are of temporary nature. Most of these approximately 900,000 cases per year, as well as many treated with geometry modifying procedures, develop arthrosis of the knee, predominantly on the medial side where the meniscus is torn either prior to or post-surgery. There is little to offer to those patients other than pain-control medications.

### Summary of the Invention

The present invention resolves the main problems of bone-sparing resurfacing surgery of the medial compartment of the knee in animals, e.g. in dogs and in people. With a hard, low abrasion, low friction coating of the implant, such as ADLC or pyrolytic carbon, it is possible to resurface only one side of the joint. In this case, the tibia plateau resurfacing with a concave recess on its femur-facing surface provides not only pain-free mobility but also the necessary stability of the joint with a deficient cruciate ligament. The planar osteotomy of the medial condyle, with or without resurfacing, can also be used to emulate TPLO procedure limited to the medial compartment of the stifle.

The hemi prosthetic replacement of the medial compartment of the knee by resurfacing the tibial condyle (frequently referred to as "plateau") disclosed in the present invention is surgically relatively simple and safe and can be performed with very simple instrumentation. The implant is dish-shaped on its upper surface, mimicking the shape of the condyle covered by the medial meniscus. This provides stability to the knee as the femoral condyle slides inside the concave recess of the implant. The surface of the implant receiving the femoral condyle is highly polished and preferably coated with a carbon-based material such as Amorphous-Diamond-Like-Carbon (ADLC) and/or pyrolytic carbon. Such coatings exhibit very low friction paired to many different materials including bone. They also result in very low wear of bone when compared to metals or ceramics. The bone-facing surface of the implant is coated for bony integration, for example with a layer of porous titanium and hydroxyapatite. Fixing means, e.g. a couple of bone screws can be used to stabilize the implant until the bone ingrowth provides sufficient interface strength.

Thus, a first aspect of the present invention is a medial tibial condyle resurfacing implant for hemiarthroplasty of the knee joint. The implant of the present invention is adapted for human medicine or veterinary medicine, e.g. for dogs.

The implant comprises an upper surface and a lower surface. In certain embodiments, the upper surface of the implant facing the femoral condyle is provided with a recess, e.g. a concave recess, for a stable articulation of the femoral condyle. The shape of the recess may be matched to the shape of the femoral condyle in the sagittal and the frontal planes. The lower surface is adapted for fixation to the condyle, e.g. adapted for boney integration by having a porous coating and/or a porous structure. In certain embodiments, the lower surface has a substantially flat shape, optionally comprising protrusions for stabilization.

The upper surface of the implant facing the femoral condyle may be coated by any suitable material, e.g. by a carbon-based material such as ADLC and/or pyrolytic carbon.

The implant of the present invention may be adapted for cementless fixation, e.g. by providing a porous coating on its bone-facing surface and/or by providing fixing means, e.g. at least two bone screws. On the other hand, the implant may also be adapted for cemented fixation.

A further aspect of the present invention relates to a medial tibial condyle osteotomy emulating procedure, in particular a TPLO procedure for stabilizing the cranial cruciate deficient stifle in a subject in need thereof, e.g. in a human or one non-human animal subject. According to this procedure, an implant according to the present invention may be placed into the knee joint of a patient in need thereof.

This invention is unique in that only the medial condyle of the tibia is resurfaced following two planar osteotomies providing easy access for fixation of the implant, yet preserving the insertion of the medial collateral ligament.

Because in most cases the femoral medial condyle will have cartilage damaged it is most important to provide low friction, low abrasion surface for the condyle of the femur to articulate against. The best options known at this time are diamond-like carbon coatings or pyrolytic carbon coatings. Of the diamond-like coatings, Amorphous Diamond-Like Carbon (ADLC) coating is very well developed and has been used in medical devices. It can be deposited on different substrates. For the present invention titanium or cobalt-chromium alloys are preferred choices as substrates. Pyrolytic carbon is usually deposited on a graphite substrate, which can also be coated for bony integration, by e.g. titanium and/or hydroxyapatite.

### List of figures

Fig. 1 shows a perspective view of the right stifle (knee) in a dog.
Fig. 2 shows a planar osteotomy of the medial condyle of the tibia.
Fig. 3 shows a second osteotomy to remove the medial plateau from the condyle.
Fig. 4 shows a medial tibial plateau replacement.
Fig. 5 shows a fixation of the plateau replacement to the condyle.
Fig. 6 shows a fixation of the condyle to the tibia.
Fig. 7 shows a TPLO emulation.
Fig. 8 shows an equivalent implant for the human knee.

### Detailed description

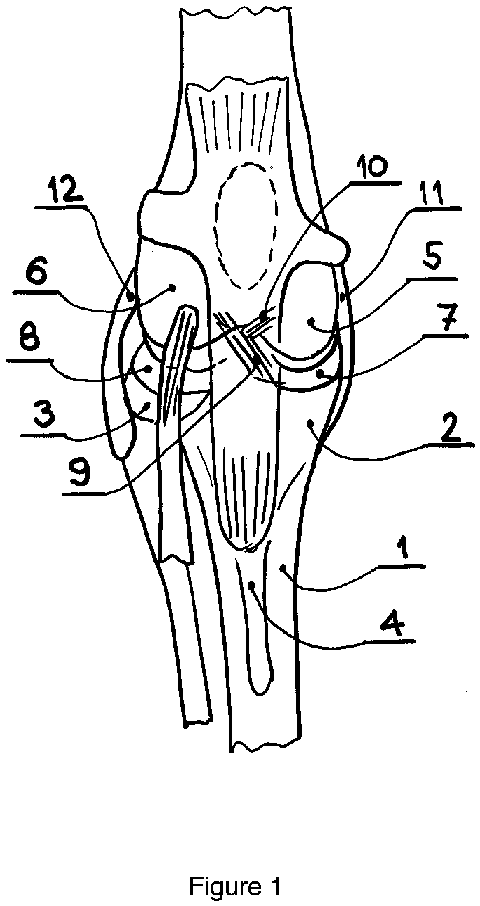

A frontal perspective view of the dog stifle (knee), Figure 1, illustrates basic anatomical features relevant to this invention. The proximal aspect of the tibia 1 flares out laterally and medially, creating a broad plateau, with the medial condyle 2 and the lateral condyle 3. Cranially to the plateau, the bone is narrowed to form the tibial tuberosity 4. Stifle joint comprises medial 5 and lateral condyles 6 of the femur, which articulate with respective condyles of the tibia. Interposed between the condyles of the femur and the tibia are menisci 7 and 8. With two bones of convex shapes, the joint is inherently unstable. Ligaments that span the joint prevent luxation. There are two main pairs: (i) cruciate ligaments 9 and 10 within the joint prevent dislocation by translation in the sagittal plane and limit the internal rotation of the tibia; (ii) collateral ligaments 11 and 12 prevent varus-valgus angulation in the frontal plane and also limit the external rotation of the tibia.

The most frequent orthopedic problem in dogs is rupture of the cranial cruciate ligament (CrCL) 9, which in human anatomy is called anterior cruciate ligament (ACL). With CrCL rupture, the femoral condyles tend to slide caudally and in doing so cause the failure of the medial meniscus and ultimately of the cartilage of both medial condyles. Surgical interventions to correct for CrCL rupture are usually performed with a delay of many weeks and months so that in about 50% of all cases treated, the medial meniscus is significantly damaged if not fully destroyed. Progression of joint degeneration is generally acknowledged if not fully accepted as a consequence of CrCL rupture, even in cases treated by complex and expensive interventions such as Tibial Plateau Leveling Osteotomy (TPLO).

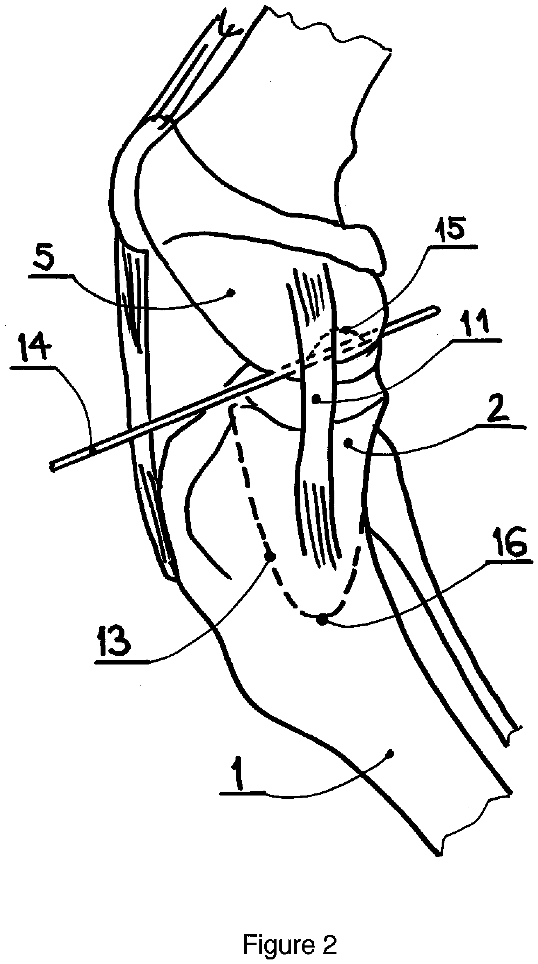

A relatively simple, planar osteotomy 13 proposed in this invention, Figure 2, allows access to the plateau of the medial tibia condyle 2 while preserving the important stabilizer of the joint - the medial collateral ligament 11. A guide wire 14 is inserted across the joint in the craniomedial to caudolateral direction, passing medially to the inter-condylar eminence 15 of the tibia. The osteotomy is fully defined by that guide wire and its exit 16 from the tibia distally. The exit 16 is located distally to the insertion of the medial collateral ligament 11.

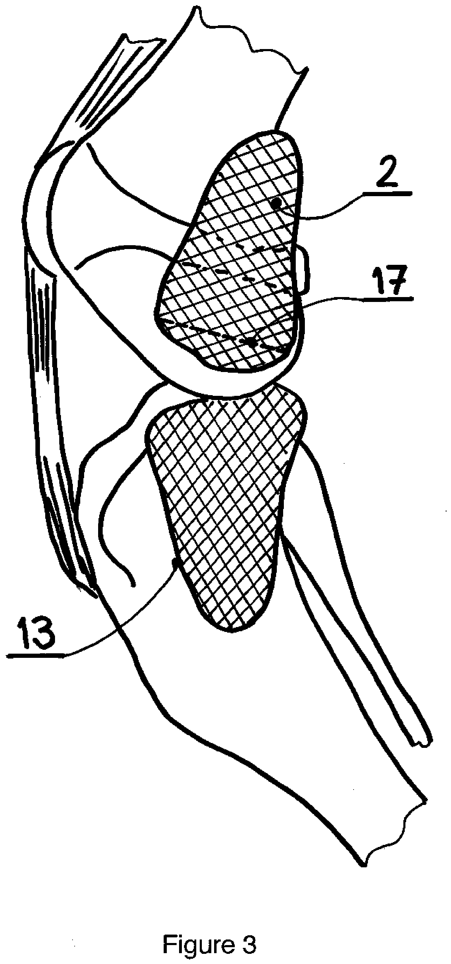

The medial condyle 2 after the first osteotomy 13 can be flipped over, Figure 3, to gain access to its proximal aspect and thus allow access for the second planar osteotomy 17, just below the subchondral bone of the condyle.

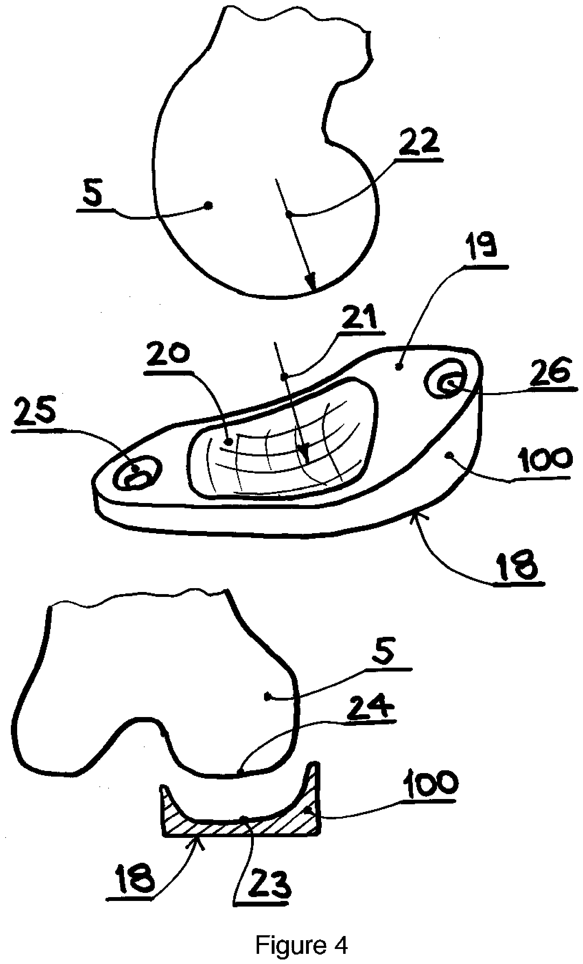

The resurfacing implant 100, Figure 4, has a flat bottom surface 18 to be affixed to the condyle 2 and an upper surface 19. The upper surface 19 has a prominent concave recess 20 to receive the femoral condyle 5 for a stable articulation. The recess 20 has a radius of curvature 21 in the sagittal plane that matches approximately the radius of the curvature 22 of the femoral condyle, also in the sagittal plane. The contour 23 of the recess 20 in the frontal plane matches the shape 24 of the femoral condyle 5 in the frontal plane. Hence the recess 20 in the upper surface of the implant 100 is shaped in the form that matches the shape of the tibial condyle covered by the meniscus. The implant 100 is provided with fixing means, e.g. at least two screw holes 25 and 26 outside the area of articulation that is limited to the recess 20. The upper surface, especially in the recess 20, may be highly polished and coated with hard, low-friction, abrasion resistant layer of e.g. ADLC or pyrolytic carbon. The lower, bone-facing surface of the implant 100 is coated for bony integration, by e.g. porous titanium and additionally by e.g. a layer of hydroxyapatite.

Alternatively, the implant 100 could be manufactured by additive manufacturing and thus provided by pores for bone integration. The upper surface 19 is fully closed, machined, polished and coated.

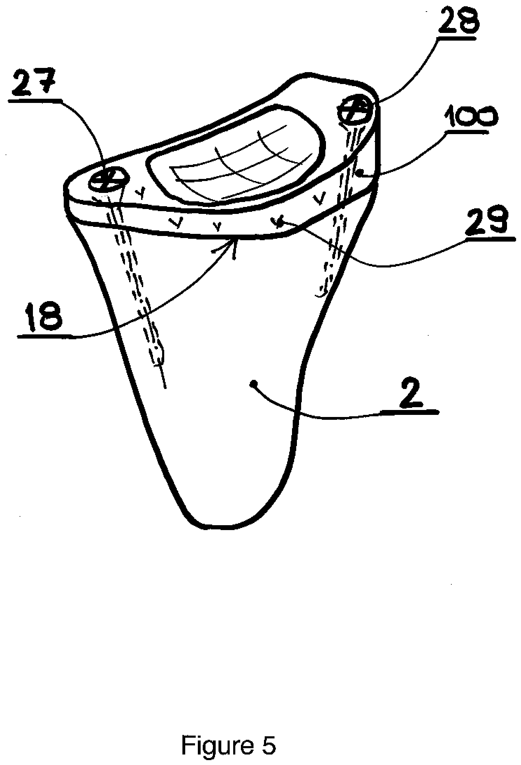

Fixation of the implant 100 to the medial condyle, Figure 5, is accomplished by placement of fixing means, e.g. at least two bone screws 27 and 28. This fixation is necessary so that bone ingrowth can proceed without movement at the interface. Further stabilization of the interface can be provided by protrusions, e.g. small spikes 29 protruding from the lower surface 18 of the implant 100. Alternatively, in special cases, such as poor cancellous bone stock, the implant 100 could be affixed to the tibia condyle by bone cement.

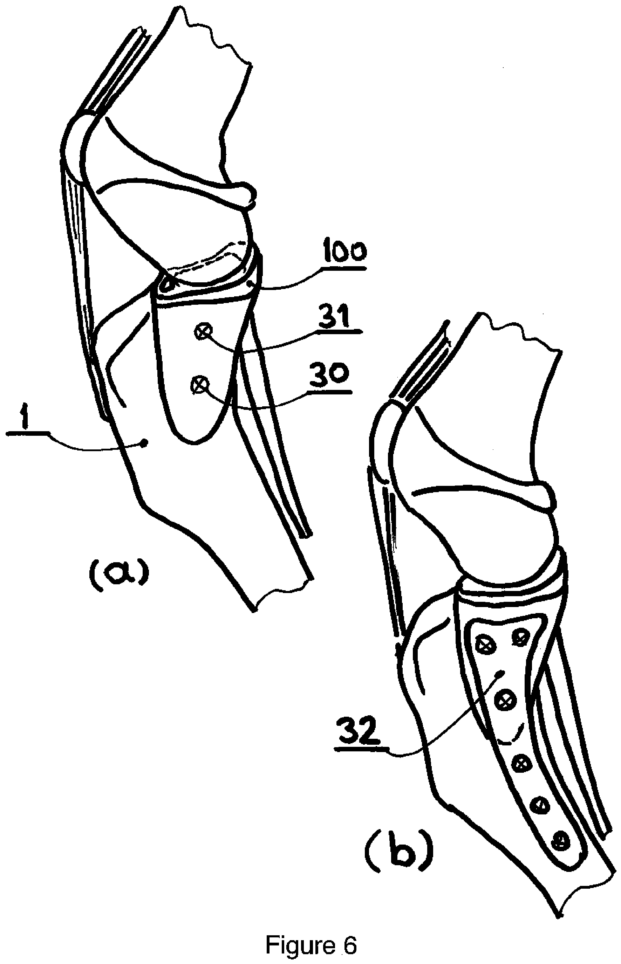

Once the implant 100 is affixed to the medial condyle 2, the condyle, together with the implant 100, can be fixed back onto the proximal aspect 1 of the tibia. Fixation by screws 30 and 31 may suffice, Figure 6a, but a bone plate 32, Figure 6b, should be used for additional stability.

Fixation of the condyle 2 to the tibia can be altered from its original position by slightly modifying its proximal-to-distal position to compensate for e.g. varus deformity. Additionally, the resurfaced tibial condyle can also be fixed back to the tibia by rotating it in the sagittal plane for added stability against caudal dislocation of the femoral medial condyle.

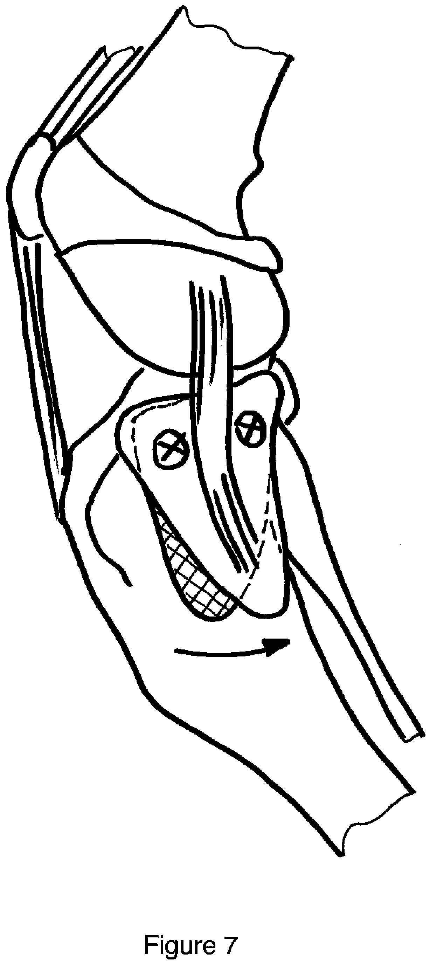

Another use of the osteotomy as disclosed here is to emulate TPLO intervention, but only on the medial compartment, Figure 7. This procedure could even be performed without the implant 100 if the condition of the medial compartment, including of the medial meniscus were satisfactory. An in-vitro test on a cadaver stifle has demonstrated the efficacy of such a reduced-morbidity TPLO on the stifle stability.

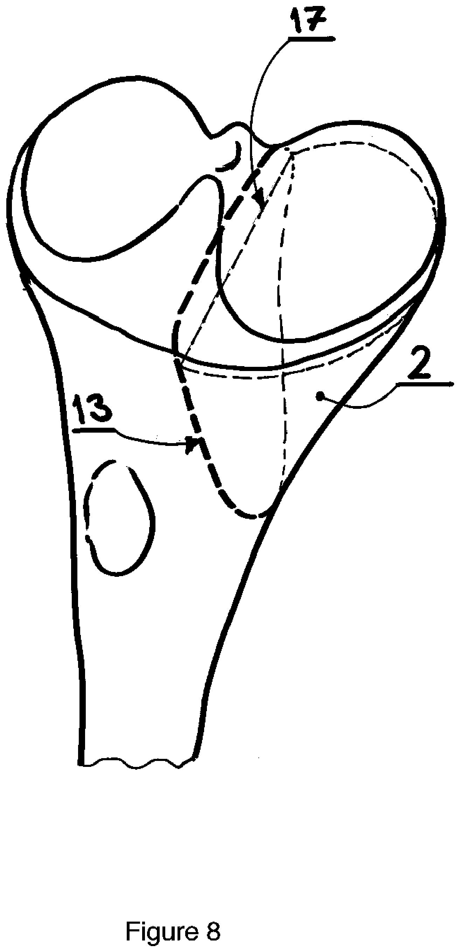

While the emphasis so far was given to the surgery on a dog, the same approach and equivalent implant design can be used for human knee, Figure 8, where two planar osteotomies 13 and 17 are performed to prepare the medial condyle 2 for fixation of the implant equivalent to that shown for dog tibia on Figures 4 and 5. The same procedure can be performed on the lateral condyle.

## Claims

1. A medial tibial condyle resurfacing implant 100 for hemiarthroplasty of the knee joint.

2. A medial tibial condyle resurfacing implant 100 of claim 1, wherein the upper surface 19 of the implant 100 facing the femoral condyle, is provided with a recess 20 for a stable articulation of the femoral condyle.

3. A medial tibial condyle resurfacing implant 100 of claim 2, wherein the shape of the recess 20 is matched to the shape of the femoral condyle in the sagittal and the frontal planes.

4. A medial tibial condyle resurfacing implant 100 of any one of claims 1-3, wherein the upper surface of the implant facing the femoral condyle is coated by a carbon-based material such as ADLC and/or pyrolytic carbon.

5. A medial tibial condyle resurfacing implant 100 of any one of claims 1-4, adapted for cementless fixation.

6. A medial tibial condyle resurfacing implant 100 of claim 5, adapted for cementless fixation by a porous coating of its bone-facing surface 18.

7. A medial tibial condyle resurfacing implant 100 of claim 5 or 6, adapted for cementless fixation secured by fixation means, e.g. at least two bone screws.

8. A medial tibial condyle resurfacing implant 100 of any one of claims 1-4, adapted for cemented fixation.

9. A medial tibial condyle resurfacing implant 100 of any one of claims 1-8 adapted for human medicine.

10. A medial tibial condyle resurfacing implant 100 of any one of claims 1-8 adapted for veterinary medicine, particularly for dogs.

11. A medial tibial condyle osteotomy emulating procedure for stabilization of the cranial cruciate deficient stifle in a subject in need thereof.
